# EUROPEAN PATENT SPECIFICATION

(11) **EP 4 291 142 B1**
(45) Date of publication and mention of the grant of the patent: **30.09.2026**
(21) Application number: 22707934.0
(22) Date of filing: 09.02.2022
(51) Int. Cl.: A61F 5/451, A61G 5/10

(54) **WHEELCHAIR SECURABLE URINE COLLECTION SYSTEMS AND RELATED METHODS**
AM ROLLSTUHL FIXIERBARE URINSAMMELSYSTEME UND ZUGEHÖRIGE VERFAHREN
SYSTÈMES DE COLLECTE D'URINE POUVANT ÊTRE FIXÉS À UN FAUTEUIL ROULANT ET MÉTHODES ASSOCIÉES

(30) Priority: 12.02.2021 US 202163148723 P
(43) Date of publication of application: 20.12.2023
(73) Proprietor: Purewick Corporation, El Cajon, California 92020 (US)
(72) Inventor: GORDON, Tacarra, Loganville, Georgia 30052 (US); JAGANNATHAN, Sudhakar, Alpharetta, Georgia 30004 (US); JARDINE, Nicholas, Holly Springs, North Carolina 27540 (US); LAI, Kuilin, Watkinsville, Georgia 30677 (US); PATEL, Puja, Lawrenceville, Georgia 30044 (US)
(74) Representative: M. Zardi & Co S.A.
(86) International application number: PCT/US2022/015781
(87) International publication number: WO 2022/173803

(56) References cited:
- JP-A- 2007 209 687
- US-A1- 2007 225 668
- US-A1- 2008 004 576
- US-A1- 2016 113 809
- US-B1- 7 491 194

## Description

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections. Conventional urine collection devices also may be limited to use when a patient is confined to a bed in a supine position.

Thus, users and manufacturers of fluid collection devices continue to seek new and improved devices, systems, and methods to collect urine. US 2007/0225668 is considered the closest prior art to the present invention. The preamble of claim 1 is based on this document. US 2016/0113809, US 2008/004576, and US 7491194 also belong to the state of the art.

### SUMMARY

Object of the present invention are a portable urine collection system according to claim 1, and a method of assembling said portable urine collection system according to claim 14. Embodiments disclosed herein are related to fluid collection devices and methods of using fluid collection devices. A portable urine collection system is disclosed. The portable urine collection system includes a urine collection device configured to be positioned at least proximate to a urethra of a user, a conduit in fluid communication with the urine collection device, a urine collection container having an interior region, a pump in fluid communication with the urine collection container, and a container support configured to detachably secure to a motorized wheelchair that is adjustable between a sitting orientation, a standing orientation, and a reclined orientation. The pump is configured to pull an at least partial vacuum on the interior region of the urine collection container effective to draw urine from the urine collection device through the first conduit into the urine collection container when the motorized wheelchair in the sitting orientation, the standing orientation, and the reclined orientation. The container support is sized and dimensioned to support the urine collection container.

A method of assembling a portable urine collection system is disclosed. The method includes detachably securing a container support to a motorized wheelchair that is adjustable between a sitting orientation, a standing orientation, and a reclined orientation. The method also includes inserting a urine collection container and a pump in fluid communication with an interior region of the urine collection container in the container support. The method also includes positioning a urine collection device proximate to a urethra of a user. The method also includes fluidly coupling the urine collection device to the urine collection container with a conduit. The pump is configured to pull an at least partial vacuum on the interior region of the urine collection container effective to draw urine from the urine collection device through the first conduit into the urine collection container and draw urine from the urine collection device when the motorized wheelchair is in each of the sitting orientation, the standing orientation, and the reclined orientation.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a portable urine collection system, according to an embodiment.
**FIG. 2** is a front view of a portable urine collection system, according to an embodiment.
**FIG. 3A** is a side view of a motorized wheelchair having a container support and in a sitting orientation, according to an embodiment.
**FIG. 3B** **is** a side view of a motorized wheelchair having a container support and in a standing orientation, according to an embodiment.
**FIG. 4** is a flow diagram of a method for assembling a portable urine collection system, according to an embodiment.
**FIG.** 5 is a block diagram of a controller in a urine collection system, according to an embodiment.

### DETAILED DESCRIPTION

Embodiments disclosed herein are related to fluid collection devices and methods of using the same. The devices and systems disclosed herein are configured to collect fluids from an individual. The fluids collected by the fluid collection devices may include at least one of urine, vaginal discharge, penile discharge, reproductive fluids, blood, sweat, or other bodily fluids. Embodiments disclosed herein are related to wheelchair mountable urine collection systems and related methods. Many users of urine collection devices are over 65 years old with limited mobility, often relying on wheelchairs as a primary mode of transportation. Many users also spend a significant amount of their day in a seated or supine position. Users and caregivers, then, are benefited from a fluid collection system that may be both discrete and mobile, allowing users to use the urine collection system to collect urine both at home and on the go.

In at least one, some, or all of the embodiments described herein, a fluid collection system is compact and includes a configuration that allows the fluid collection system to be secured or securable (e.g., mounted or mountable) to a wheelchair. At least one, some, or all of the embodiments of the fluid collection systems described herein have a configuration resulting in the technical effect the fluid collection system being mobile and discreet, allowing a user to participate in social activities without alerting others to the incontinence of the users. In at least one, some, or all of the embodiments of fluid collection systems described herein, an alert system is included that is configured to communicate with an electronic device, resulting in the technical effect of alerting a user or caregiver to empty the fluid collection container when the fluid level approaches a predetermined level, to change or recharge a battery, and/or adjust a vacuum or suction level of the pump in the fluid collection system.

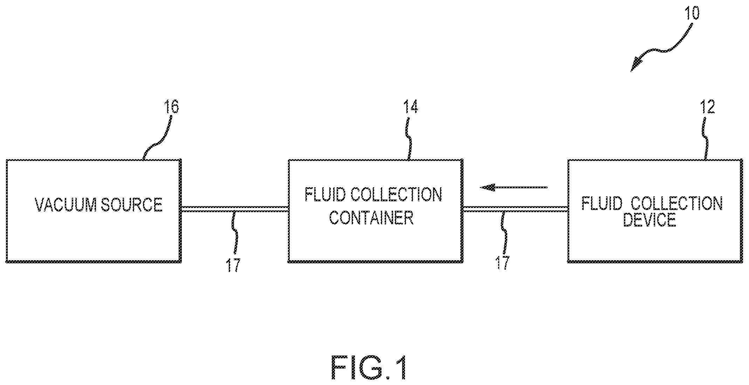

**FIG. 1** is a block diagram of a fluid collection system 10, according to an embodiment. The fluid collection system 10 may be included in embodiments of fluid collection systems described herein. The system 10 includes a fluid collection device 12 *(e.g.,* any of the fluid collection assemblies disclosed herein), a fluid collection container 14, and a vacuum source 16 (or pump). The fluid collection device 12, the fluid collection container 14, and the vacuum source 16 may be fluidly coupled to each other via one or more conduits 17. For example, fluid collection device 12 may be operably coupled to one or more of the fluid collection container 14 or the vacuum source 16 via the conduit 17. In some embodiments, the vacuum source 16 may be secured directly to the fluid collection container 14. Fluid *(e.g.,* urine or other bodily fluids) collected in the fluid collection device 12 may be removed from the fluid collection device 12 via the conduit 17 secured to the fluid collection device 12. Suction force may be introduced into the chamber of the fluid collection device 12 via the inlet of the conduit 17 responsive to suction (e.g., vacuum) force applied at the outlet of the conduit 17.

The suction force may be applied to the outlet of the conduit 17 by the vacuum source 16 either directly or indirectly. The suction force may be applied indirectly via the fluid collection container 14. For example, the outlet of the conduit 17 may be disposed within or fluidly coupled to an interior region of the fluid collection container 14 and an additional conduit 17 may extend from the fluid collection container 14 to the vacuum source 16. Accordingly, the vacuum source 16 may apply suction to the fluid collection device 12 via the fluid collection container 14. The suction force may be applied directly via the vacuum source 16. For example, the outlet of the conduit 17 may be disposed within the vacuum source 16. An additional conduit 17 may extend from the vacuum source 16 to a point outside of the fluid collection device 12, such as to the fluid collection container 14. In such examples, the vacuum source 16 may be disposed between the fluid collection device 12 and the fluid collection container 14.

The fluid collection container 14 is sized and shaped to retain a fluid therein. The fluid collection container 14 may include a bag (e.g., drainage bag), a bottle or cup *(e.g.,* collection jar), or any other enclosed container for storing bodily fluid(s) such as urine. In some examples, the conduit 17 may extend from the fluid collection device 12 and attach to the fluid collection container 14 at a first point therein. An additional conduit 17 may attach to the fluid collection container 14 at a second point thereon and may extend and attach to the pump 16. Accordingly, a vacuum (e.g., suction) may be drawn through fluid collection device 12 via the fluid collection container 14. Fluid, such as urine, may be drained from the fluid collection device 12 using the vacuum source 16.

The vacuum source 16 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 16 may provide a vacuum or suction to remove fluid from the fluid collection device 12. In some examples, the vacuum source 16 may be powered by one or more of a power cord *(e.g.,* connected to a power socket), one or more batteries, or even manual power *(e.g.,* a hand operated vacuum pump). In some examples, the vacuum source 16 may be sized and shaped to fit outside of, on, or within the fluid collection device 12. For example, the vacuum source 16 may include one or more miniaturized pumps or one or more micro pumps. The vacuum sources disclosed herein may include one or more of a switch, a button, a plug, a remote, or any other device suitable to activate the vacuum source 16.

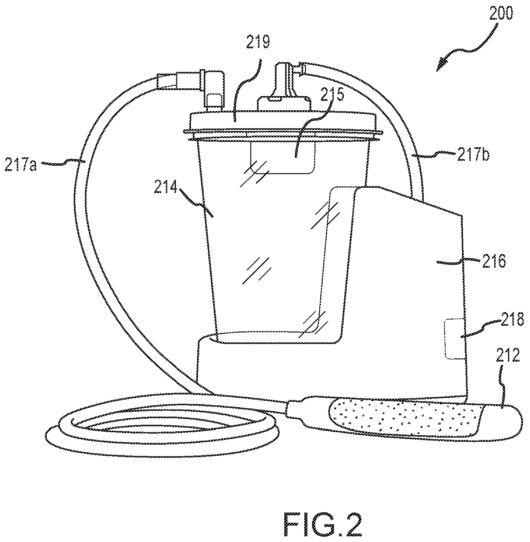

**FIG. 2** shows an example of a urine collection system 200, according to an embodiment. While urine collection is referenced with respect to the urine collection system 200, the urine collection system 200 may collect other bodily fluids, such as vaginal discharge, penile discharge, reproductive fluids, blood, and/or sweat. The urine collection system 200 may include a urine collection device 212, a first conduit 217a in fluid communication with an interior region of the urine collection device 212, a urine collection container 214 having an interior region in fluid communication with the internal region of the urine collection device 212 via the first conduit 217a, a second conduit 217b in fluid communication with the interior region of the urine collection container 214, a pump 216 (e.g., vacuum source) in fluid communication with the interior region of the urine collection container 214 via the second conduit 217b, and a sensor 215. The urine collection device 212 is configured to be positioned at least proximate to a urethra of a user. While the urine collection device 212 shown in FIG. 2 includes a female urine collection device, the urine collection device 212 may instead include a male urine collection device. PCT International Application No. PCT/US2019/029616, for example, describes various embodiments of both male and female fluid collection devices, to the disclosure of which reference is herein made. Moreover, the urine collection device 212 may be interchangeable in the urine collection system 200 between different types, varieties, and sizes of male or female urine collection devices or other fluid collection devices. Generally, the urine collection device 212 may include a surface sized to be positioned proximate or adjacent to the urethra and configured to wick urine or other fluids away from the user. Urine or other fluids may be wicked from the surface to a reservoir in the urine collection device 212.

The urine collection system 200 also includes a first conduit 217a in fluid communication with an interior region (e.g. reservoir) of the urine collection device 212 and an interior region of the urine collection container 214. The first conduit 217a may be positioned between the urine collection device 212 and the urine collection container 214. The urine collection system 200 also may include a second conduit 217b providing fluid communication between the pump 216 and the interior region of the urine collection container 214. In some embodiments, the pump 216 may be secured directly to the urine collection container 214, and the second conduit 217b may be absent from the urine collection system 200. The conduits 217a, 217b may include a flexible tube. In some embodiments, at least a portion of the first conduit 217a is substantially opaque, thereby inhibiting viewing of the urine within the first conduit 217a.

The urine collection container 214 may be reusable and dishwasher safe, and may include a generally rigid material such as polycarbonate or glass. In some embodiments, the urine collection container 214 may be disposable. The urine collection system 200 and a container support 302 may be configured to accommodate different volumetric sizes of urine collection containers 214. In some embodiments, a lid 219 having multiple ports and/or adapters for attachment of the conduits 217a, 217b thereto may be secured or securable to the urine collection container 214. Except for the ports and/or adapters for attachment of the conduits 217a, 217b, the urine collection container 214 may be sealed and airtight effective resulting in the technical effect of preventing the urine collected in the urine collection container 214 from leaking or spilling when the wheelchair 300 changes orientation.

The urine collection system also may include a sensor 215. The sensor 215 is secured to the urine collection container 214 and/or the lid 219 and configured to detect a property relating at least to a volume of the urine in the interior region of the urine collection container 214, according to an embodiment. In some embodiments, the sensor 215 includes an ultrasonic sensor, a laser sensor, a wet sensor, a water sensor, a negative pressure sensor, a weight sensor, or an ultraviolet (UV) sensor. In at least one, some, or all of the embodiments of the urine collection system 200, the sensor 215 may be provides the technical effect of a continuous or periodical feedback of the property relating at least to a volume of the urine with or without the sensor 215 touching the urine in the urine collection container 214. The sensor 215 may be secured to the urine collection container 214 or the lid 219 with at least a portion of the sensor 215 inside the interior region of the urine collection container 214. In some embodiments, the sensor 215 is positioned at a top of the urine collection container 214 and pointed downwards where the urine collects in the interior region of the urine collection container 214.

In some embodiments, the sensor 215 includes a weight sensor secured to one of the urine collection container 214 or the container support 302 and configured to detect a weight of the volume of urine collected in the urine collection container 214. In some embodiments, the sensor 215 includes a level transmitter configured to detect a level of the urine in the urine collection container 214. The sensor 215 may include a laser level sensor that measures the distance between the laser transmitter of the sensor 215 to a surface of the urine and back to a detector of the sensor 215. The elapsed time between transmitting the laser and detecting the reflected laser may be measured by the sensor 215 and a distance between the sensor 215 and the level of the urine may be calculated by the sensor 215 or an associated controller. The laser may be transmitted through a clear window on the urine collection container 214 or at least the transmitter of the sensor 215 may be positioned within the urine collection container 214.

In some embodiments, the sensor 215 may include an ultrasonic level sensor that leverages the speed of sound to detect the property relating at least to a volume of the urine in the urine collection container 214. For example, the sensor 215 may measure a distance between a transducer of the sensor 215 and a surface of the urine in the urine collection container 214 using a time for the pulse to travel from the transducer to the surface of the urine and back to the transducer of the sensor 215. The sensor 215 or a controller associated with the sensor 215 may determine a volume of the urine in the urine collection container 214 using the distance between the sensor 214 and the surface of the urine in the urine collection container 214.

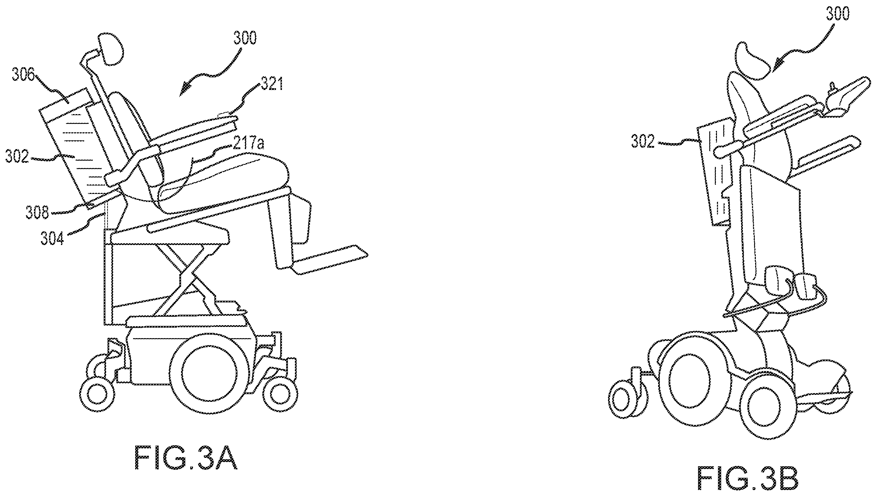

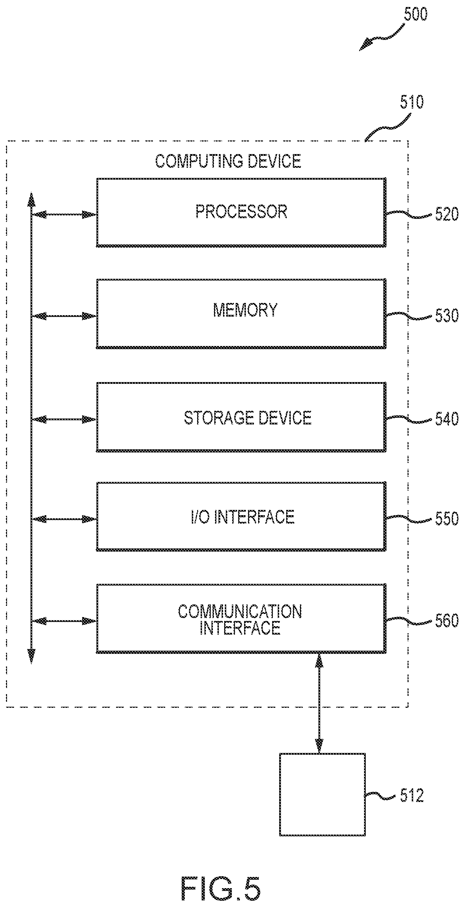

In some embodiments, the sensor 215 also includes a controller. The controller may be included with the sensor 215 or may be a separate component configured to communicate with the sensor 215. For example, the sensor 215 may include a communication interface configured to communicate with a controller 321 (shown in **FIG. 3A**) mounted on the wheelchair or the container support 302. The controller may include a processor configured to calculate a level or volume of urine in the urine collection container 214. The sensor 215 and/or the controller may include a communication interface configured to send notifications or alerts to other electronic devices. For example, the communication interface may be configured to send notifications or alerts at a selected radio frequency, via BLUETOOTH, or via WI-FI to another electronic device, such as a mobile phone of the user or caregiver. The sensor 215 and/or the controller may be powered by an external or internal battery, such as a rechargeable battery. **FIG. 5** provides additional details of a controller 500 that may include the controller of the urine collection system 200.

In at least one, some, or all of the embodiments of the urine collection system 200, the configuration of the controller results in the technical effect of wirelessly transmitting one or more alerts to at least one of an electronic device of the user or a caregiver. For example, the controller may be configured to transmit an alert to an electronic device of the user or a caregiver when the property relating at least to the volume of the urine detected by the sensor 215 indicates the volume of the urine in the urine collection container 215 has reached or exceeded a predetermined volume. For example, based on data from the sensor 215, the controller may wirelessly transmit an alert to an electronic device and/or coordinate an alert to notify the user or the caregiver that the urine in the urine collection device is a predetermined distance (such as about 2.5 cm) from the sensor 215 and/or contacting the sensor 215, and emptying of the urine collection container 214 is recommended. In some embodiments, the controller may wirelessly transmit and/or coordinate alerts and selected frequencies, such as selected time and/or volume intervals. In some embodiments, the controller is configured to coordinate an alert to notify the user or the caregiver that the volume of the urine in the urine collection container 214 has reached or exceeded two or more of 50% of a maximum volume of the urine collection container, 75% of the maximum volume of the urine collection container, and/or 90% of the maximum volume of the urine collection container.

In some embodiments, the controller may wirelessly transmit an alert to the electronic device of the user or the caregiver and/or coordinate a battery charging alert to notify the user or the caregiver that charging of the rechargeable battery powering at least one of the pump 216 or the controller is suggested. The controller may wirelessly transmit an alert to the electronic device of the user or the caregiver and/or coordinate an alert to notify the user or the caregiver when replacement of a filter (such as the filter 218) of the urine collection system 200 is recommended. In some embodiments, the controller may wirelessly transmit a solar panel alert to the electronic device of the user or the caregiver and/or coordinate a solar panel alert indicating performance of the solar panel. In some embodiments, the controller may wirelessly transmit a position alert to the electronic device of the user or the caregiver and/or coordinate a position alert indicating a change of position by the user is suggested. Alerts coordinated by the controller may include one or more of a light, a noise, and/or a haptic feedback.

In at least one, some, or all of the embodiments of the urine collection system 200, a filter 218 is included that results in the technical effect of neutralizing and/or enhancing (e.g., improving) odor of the air being pulled from the interior region of the urine collection container 214 by the pump 216. In some embodiments, the filter 218 is positioned on an exhaust vent on the pump 216. In some embodiments, the filter 218 is positioned between at least a portion of the conduit 217b and a portion of the interior region of the urine collection container 214 such that air being pulled from the interior region of the urine collection container 214 is filtered before or as the air enters the conduit 217b. In some embodiments, a filter 218 is positioned both at the exhaust vent on the pump 216 and before air enters the conduit 217b. In some embodiments, the filter 218 may be positioned at least partially within the second conduit 217b and/or throughout one or more portions of the second conduit 217b. The filter 218 may include an odor absorbing filter, such as activated carbon and/or zeolite filters.

In some embodiments, the filter 218 may include an aromatherapy pack or an aromatherapy pack may be secured proximate to the exhaust vent of the pump to produce a more pleasant smell. The filter 218 may include baking soda or other composition that removes odor from the air and/or adds pleasant aroma to the air. In some embodiments, the filter 218 is absent and the pump 216 includes the aromatherapy pack removably secured to the pump 216 proximate to the exhaust vent.

In at least one, some, or all embodiments of the urine collection system 200, the pump 216 is in fluid communication with the interior region of the urine collection container 214 and is configured to pull a vacuum on the interior region of the urine collection container 214 effective to draw the urine from the urine collection device 212 through the first conduit 217a into the urine collection container 214. The pump 216 may be secured directly to the urine collection container 214, or the conduit 217b may fluidly couple the pump 216 with the interior region of the urine collection container.

The pump 216 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 216 may provide a vacuum or suction to remove fluid from the fluid collection device 212. In some examples, the pump 216 may be powered by one or more batteries or other power sources. In some examples, the pump 216 may be sized and shaped to fit within the container support 302. The pump 216 may include one or more of a switch, a button, a plug, a remote, or any other device suitable to activate the pump 216.

**FIGS. 3A** and **3B** show a container support 302 of a urine collection system mounted to a wheelchair 300. The container support 302 is configured to be mounted to a motorized wheelchair 300 and support the urine collection container 214 and/or the pump 216 therein when secured to the motorized wheelchair 300. The container support 302 includes one or more rigid walls. For example, the container support 302 may include at least a rigid bottom wall configured to support at least the urine collection container 214. The container support 302 includes one or more side walls that, in some embodiments, also are generally rigid or are generally flexible. For example, the container support 302 may include one more side walls that are generally flexible and/or adjustable effective to allow an internal volume of the container support to be adjusted. Rigid walls of the container support 302 may include plastic, metal, word, composite materials, or combinations thereof. In some embodiments, rigid walls of the container support 302 may include a lining material or fabric.

The container support 302 includes a top wall or lid that is generally rigid or flexible. The top wall or lid is adjustable between an open position and a closed position. For example, in an open position, the top wall or lid may be connected to a sidewall via one or more hinges or connecting material. When the top wall or lid is in the open position, a user or caregiver may insert and remove the urine collection container 214 and/or the pump into the container support 302. The top wall or lid may include a locking mechanism such as a latch, clamp, clip, or zipper configured to selectively retain the top wall or lid in the closed position.

In at least one, some, or all embodiments, the container support 302 includes one or more sound dampening elements such as foam and/or sound dampening fabric on the interior surface of one or more walls of the container support 302 resulting in the technical effect of reducing or eliminating operational noises (such as the pump 216 running and/or splashing of urine) of the urine collection system 200 emanating from the container support 302. In at least one, some, or all embodiments, the container support 302 may include a window in a sidewall or the top wall resulting in the technical effect of allowing a user or caregiver to view a urine level in the urine collection container 214. The sidewalls may otherwise be opaque effective to obscure undesirable viewing of the urine collection container 214 housed in the container support 302.

The container support 302 may be mounted to a motorized wheelchair 300 that is adjustable between a standing orientation and a sitting orientation. **FIG. 3A** shows the wheelchair 300 in the sitting orientation and **FIG. 3B** shows the wheelchair in the standing orientation. In some embodiments, the container support 302 may be mounted to a motorized wheelchair 300 that is adjustable between a standing orientation, a sitting orientation, and/or a reclined or lay flat orientation. When the pump 216 and urine collection container 214 are positioned within the container support 302, the urine collection system 200 may collect and temporarily store urine from a user of the wheelchair 300 when the wheelchair 300 is in any of the standing orientation, the sitting orientation, and/or the reclined orientation. A urine collection system that is mountable to a wheelchair 300 having a standing orientation is beneficial because standing reduces pressure on internal organs of the user and improves lung volume, which increases blood oxygenation in the user. Standing also reduces edema, the risk of deep vein thrombosis, and the risk of urinary tract infections in the user

The container support 302 is detachably mounted or secured to the back of the wheelchair 300. In some embodiments, the container support 302 is mechanically mounted to wheelchair 300 with one or more fasteners, straps, support arms, clamps, clips, or any combinations thereof. The container support 302 is sized and dimensioned to house and/or support the urine collection container 214 and the pump 216 therein. The container support 302 may be sized and dimensioned to house and/or support urine collection containers and pumps of other urine collection systems therein. The container support 302 may include a hole or opening in the top or a sidewall sized and dimensioned to extend a portion of the first conduit 217a through the container support 302 to allow urine to be transferred from the urine collection device outside the container support 302 to the urine collection container 214 inside the container support 302. The container support 302 includes a port or adapter configured to attach or secure to the first conduit 217a. The urine collection system 200 also includes an additional conduit positioned in the container support 302 providing fluid communication between the first conduit 217a and the urine collection container 214. Accordingly, the first conduit 217a attaches to the port or adapter on the container support 302 on the outside of the container support 302 and the additional conduit attaches to the port or adapter on the container support on the inside of the container support 302, with the port or adapter providing fluid communication between the first conduit 217a and the additional conduit housed in the container support 302.

In some embodiments, the urine collection system 200 may include a rechargeable battery 308 electrically connected to the pump 216 and configured to power the pump 216. The rechargeable battery 308 may be removably mounted or secured to the container support 302. In some embodiments, the rechargeable battery 308 may be positioned within the container support 308 and not visible outside the container support 302. For example, the rechargeable battery 308 may be mounted within the container support 302 or mounted to the pump 216. In some embodiments, the rechargeable battery 308 may be electrically coupled to the battery or power source of the wheelchair 300 with an electrical cable 304. The electrical cable 304 also may be configured to connect to a wall outlet for charging the rechargeable battery. In some embodiments, the electrical cable 304 electrically couples the pump 216 with the battery or power source of the wheelchair 300.

In some embodiments, the container support 302 includes one or more solar panels 306 secured to an outer portion of the container support 302 and electrically coupled to at least one of the rechargeable battery 308 and/or the pump 216. The one or more solar panels 306 may be configured to at least partially recharge the rechargeable battery 308 and/or power the pump 216. In some embodiments, a controller 321 may be secured to the arm or other member of the wheelchair 300. The controller 321 may include a touch screen and may include any aspect of the controller described above in relation to the urine collection system 200.

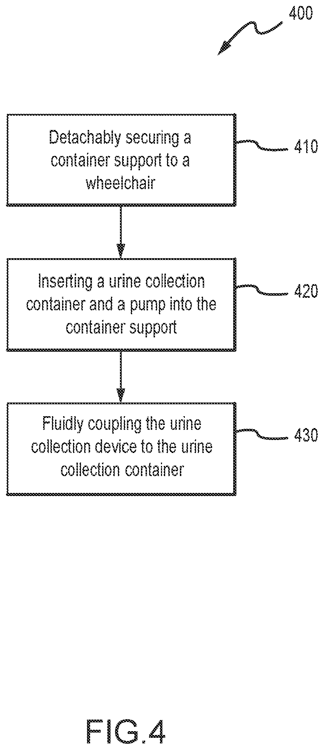

**FIG. 4** is a flow diagram of a method 400 for assembling a portable urine collection system, according to an embodiment. The method includes an act 410 of detachably securing a container support to a motorized wheelchair that is adjustable between a sitting orientation, a standing orientation, and a reclined orientation. In some embodiments, the act 410 includes detachably securing the container support to a back of the motorized wheelchair. In some embodiments, the act 410 includes detachably securing the container support underneath a seat of the wheelchair.

The method 400 may include an act 420 of inserting a urine collection container and a pump in fluid communication with an interior region of the urine collection container in the container support. In some embodiments, the method 400 also includes an act inserting one or more sound dampening elements into the container support before or after the urine collection container and the pump are inserted into the container support. In some embodiments, the method 400 also includes an act of positioning a urine collection device proximate to a urethra of a user.

The method 400 may include an act 430 of fluidly coupling the urine collection device to the urine collection container with a first conduit. The pump may be configured to pull a vacuum on the interior region of the urine collection container effective to draw urine from the urine collection device through the first conduit into the urine collection container and draw urine from the urine collection device when the motorized wheelchair is in each of the sitting orientation, the standing orientation, and the reclined orientation.

In some embodiments, the method 400 also includes an act of electrically coupling a solar panel on the outer portion of the container support to a rechargeable battery that is electrically coupled to the pump. The method may include electrically coupling the rechargeable battery to one or more of a power source of the motorized wheelchair or an electrical outlet with a cable to recharge the rechargeable battery or power the pump. In some embodiments, the method includes electrically coupling the pump to a battery or power source of the motorized wheelchair with a cable to power the pump.

In some embodiments, the method 400 includes an act of activating a sensor secured to one of the urine collection container or the container support to detect a property relating at least to a volume of the urine in the urine collection container. The method 400 may include an act of activating a controller to communicate with the sensor and coordinate a user alert when the volume of the urine in the urine collection container has reached or exceeded a predetermined volume. The controller may be configured coordinate a user alert to notify the user or a caregiver that the volume of the urine in the urine collection container has reached or exceeded the predetermined volume. The method 400 may include an act of activating the controller to coordinate the user alert to notify the user or the caregiver that the volume of the urine in the urine collection container has reached or exceeded two or more of 50% of a maximum volume of the urine collection container, 75% of the maximum volume of the urine collection container, or 90% of the maximum volume of the urine collection container. The method 400 may include an act of activating the controller to coordinate at least one of a filter replacement alert indicating replacement of the one or more activated carbon and zeolite filters is suggested, a battery charging alert indicating charging of the rechargeable battery is suggested, a battery replacement alert indicating replacement of the rechargeable battery is suggested, a solar panel alert indicating performance of the solar panel, or a position alert indicating a change of position by the user is suggested. In some embodiments, the method includes securing the controller to an arm of the motorized wheelchair.

The acts of the method 400 described above are for illustrative purposes. For example, the acts of the method 400 can be performed in different orders, split into multiple acts, modified, supplemented, or combined. In an embodiment, one or more of the act of the method 400 can be omitted from the method 400. Any of the acts of the method 400 can include using any of the portable urine collection systems disclosed herein.

**FIG. 5** is a schematic of a controller 500 that may be used with any of the systems and methods described herein, according to an embodiment. For example, the controller of the urine collection system 200 and/or the controller 321 may include any aspect of the controller 500. The controller 500 may be configured to implement any of the example acts or steps disclosed herein, such as wired or wirelessly communicating with the sensor 215 or other electronic devices, determining a volume of the urine in the urine collection container 214, and/or transmitting and/or coordinating alerts described herein.

The controller 500 includes at least one computing device 510, according to an embodiment. The at least one computing device 510 is an exemplary computing device that may be configured to perform one or more of the acts described above. The computing device 510 can comprise at least one processor 520, memory 530, a storage device 540, an input/output ("I/O") device/interface 550, and a communication interface 560. While an example computing device 510 is shown in **FIG. 5****,** the components illustrated in **FIG. 5** are not intended to be limiting of the controller 500 or computing device 510. Additional or alternative components may be used in some examples. Further, in some examples, the controller 500 or the computing device 510 can include fewer components than those shown in **FIG. 5****.** For example, the controller 500 may not include the one or more additional computing devices 512. In some examples, the at least one computing device 510 may include a plurality of computing devices. Components of computing device 510 shown in **FIG. 5** are described in additional detail below.

In some examples, the processor(s) 520 includes hardware for executing instructions (e.g., instructions for carrying out one or more portions of any of the methods disclosed herein), such as those making up a computer program. For example, to execute instructions, the processor(s) 520 may retrieve (or fetch) the instructions from an internal register, an internal cache, the memory 530, or a storage device 540 and decode and execute them. In particular examples, processor(s) 520 may include one or more internal caches for data such as tables pertaining to volumetric tables. As an example, the processor(s) 520 may include one or more instruction caches, one or more data caches, and one or more translation lookaside buffers (TLBs). Instructions in the instruction caches may be copies of instructions in memory 530 or storage device 540. In some examples, the processor 520 may be configured (e.g., include programming stored thereon or executed thereby) to carry out one or more portions of any of the example methods disclosed herein.

In some examples, the processor 520 is configured to perform any of the acts disclosed herein or cause one or more portions of the computing device 510 or controller 500 to perform at least one of the acts disclosed herein. Such configuration can include one or more operational programs (e.g., computer program products) that are executable by the at least one processor 520. For example, the processor 520 may be configured to automatically determine a volume of urine in a urine collection container, automatically determine a proximity of urine in the urine collection container to a sensor, automatically transmit an alert when the volume of the urine in the urine collection container meets or exceeds a predetermined threshold, automatically transmit an alert when a change of filter is suggested, and/or automatically transmit an alert when a change or recharge of battery is suggested.

The at least one computing device 510 (e.g., a server) may include at least one memory storage medium (e.g., memory 530 and/or storage device 540). The computing device 510 may include memory 530, which is operably coupled to the processor(s) 520. The memory 530 may be used for storing data, metadata, and programs for execution by the processor(s) 520. The memory 530 may include one or more of volatile and non-volatile memories, such as Random Access Memory (RAM), Read Only Memory (ROM), a solid state disk (SSD), Flash, Phase Change Memory (PCM), or other types of data storage. The memory 530 may be internal or distributed memory.

The computing device 510 may include the storage device 540 having storage for storing data or instructions. The storage device 540 may be operably coupled to the at least one processor 520. In some examples, the storage device 540 can comprise a non-transitory memory storage medium, such as any of those described above. The storage device 540 (e.g., non-transitory storage medium) may include a hard disk drive (HDD), a floppy disk drive, flash memory, an optical disc, a magneto-optical disc, magnetic tape, or a Universal Serial Bus (USB) drive or a combination of two or more of these. Storage device 540 may include removable or non-removable (or fixed) media. Storage device 540 may be internal or external to the computing device 510. In some examples, storage device 540 may include non-volatile, solid-state memory. In some examples, storage device 540 may include read-only memory (ROM). Where appropriate, this ROM may be mask programmed ROM, programmable ROM (PROM), erasable PROM (EPROM), electrically erasable PROM (EEPROM), electrically alterable ROM (EAROM), or flash memory or a combination of two or more of these. In some examples, one or more portions of the memory 530 and/or storage device 540 (e.g., memory storage medium(s)) may store one or more databases thereon.

In some examples, one or more of a history of the volume of the urine in the urine collection container, a trend of the volume of the urine in the urine collection container, a history of filter replacement, and/or a history of battery replacement or recharging may be stored in a memory storage medium such as one or more of the at least one processor 520 (e.g., internal cache of the processor), memory 530, or the storage device 540. In some examples, the at least one processor 520 may be configured to access (e.g., via bus 570) the memory storage medium(s) such as one or more of the memory 530 or the storage device 540. For example, the at least one processor 520 may receive and store the data (e.g., look-up tables) as a plurality of data points in the memory storage medium(s). The at least one processor 520 may execute programming stored therein adapted access the data in the memory storage medium(s) to automatically determine a volume of urine in a urine collection container, automatically determine a proximity of urine in the urine collection container to a sensor, automatically transmit an alert when the volume of the urine in the urine collection container meets or exceeds a predetermined threshold, automatically transmit an alert when a change of filter is suggested, and/or automatically transmit an alert when a change or recharge of battery is suggested. For example, the at least one processor 520 may access one or more look-up tables in the memory storage medium(s) such as memory 530 or storage device 540.

The computing device 510 also includes one or more I/O devices/interfaces 550, which are provided to allow a user to provide input to, receive output from, and otherwise transfer data to and from the computing device 510. These I/O devices/interfaces 550 may include a mouse, keypad or a keyboard, a touch screen, camera, optical scanner, network interface, web-based access, modem, a port, other known I/O devices or a combination of such I/O devices/interfaces 550. The touch screen may be activated with a stylus or a finger.

The I/O devices/interfaces 550 may include one or more devices for presenting output to a user, including, but not limited to, a graphics engine, a display (e.g., a display screen or monitor), one or more output drivers (e.g., display drivers), one or more audio speakers, and one or more audio drivers. In certain examples, I/O devices/interfaces 550 are configured to provide graphical data to a display for presentation to a user. The graphical data may be representative of one or more graphical user interfaces and/or any other graphical content as may serve a particular implementation.

The computing device 510 can further include a communication interface 560. The communication interface 560 can include hardware, software, or both. The communication interface 560 can provide one or more interfaces for communication (such as, for example, packet-based communication) between the computing device 510 and one or more additional computing devices 512 or one or more networks. For example, communication interface 560 may include a network interface controller (NIC) or network adapter for communicating with an Ethernet or other wire-based network or a wireless NIC (WNIC) or wireless adapter for communicating with a wireless network, such as a WI-FI. The one or more additional computer device 512 may include a smart phone of the user, a smart phone of the caregiver, an electronic device on the wheelchair, and/or a computer device of a healthcare system.

Any suitable network and any suitable communication interface 560 may be used. For example, computing device 510 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, one or more portions of controller 500 or computing device 510 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination thereof. The computing device 510 may include any suitable communication interface 560 for any of these networks, where appropriate.

The computing device 510 may include a bus 570. The bus 570 can include hardware, software, or both that couples components of computing device 510 to each other. For example, bus 570 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus,
a PCI-Express (PCIe) bus, a serial advanced technology attachment (SATA) bus, a Video Electronics Standards Association local (VLB) bus, or another suitable bus or a combination thereof.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting. The invention is defined by the claims solely.

## Claims

1. A portable urine collection system, comprising:
a motorized wheelchair (300);
a urine collection device (212) configured to be positioned at least proximate to a urethra of a user;
a first conduit (217a) in fluid communication with the urine collection device;
a urine collection container (214) having an interior region;
a pump (216) in fluid communication with the urine collection container and configured to pull an at least partial vacuum on the interior region of the urine collection container effective to draw urine from the urine collection device through the first conduit into the urine collection container; and
a container support (302) including one or more sidewalls and a lid adjustable between an open position and a closed position, the container support being mechanically secured to a back of the motorized wheelchair (300) to detachably secure the container support to the motorized wheelchair, wherein the container support is sized and dimensioned to support the urine collection container and the pump therein, and the pump is configured to draw the urine from the urine collection device when the motorized wheelchair is in each of the sitting orientation, the standing orientation, and the reclined orientation;
said portable urine collection system being **characterized in that** said motorized wheelchair is adjustable between a sitting orientation, a standing orientation, and a reclined orientation; the container support (302) including a port or adapter configured to attach or secure to the first conduit (217a); the urine collection system including an additional conduit positioned in the container support (302) providing fluid communication between the first conduit (217a) and the urine collection container (214); the first conduit (217a) being attached to the port or adapter on the container support (302) on the outside of the container support (302) and the additional conduit being attached to the port or adapter on the container support on the inside of the container support (302) with the port or adapter providing fluid communication between the first conduit (217a) and the additional conduit housed in the container support (302).

2. The portable urine collection system of claim 1, wherein the container support (302) includes one or more sound dampening elements disposed on an interior surface of the one or more sidewalls of the container support, the one or more sound dampening elements including at least one of foam or sound dampening fabric.

3. The portable urine collection system of any of claims 1-2, wherein the lid (219) of the container support (302) includes a locking mechanism configured to selectively retain the lid in the closed position.

4. The portable urine collection system of any of claims 1-3, further comprising:
a rechargeable battery (308) electrically coupled to the pump (216) and configured to power the pump;
a solar panel (306) secured to an outer portion of the container support (302) and electrically coupled to the rechargeable battery, the solar panel configured to at least partially recharge the rechargeable battery; and
a cable (304) electrically coupled to the rechargeable battery and coupled to one or more of a power source of the motorized wheelchair or an electrical outlet to recharge the rechargeable battery or power the pump.

5. The portable urine collection system of any of claims 1-3, further comprising a cable (304) electrically coupled to the pump (216) and to a battery or a power source (308) of the motorized wheelchair (300) to power the pump.

6. The portable urine collection system of any of claims 1-5, further comprising:
a second conduit (217b) providing fluid communication between the urine collection container (214) and the pump (216); and
one or more activated carbon and zeolite filters (218) configured to remove odors from air passing therethrough, the one or more activated carbons being positioned in the second conduit, between the interior region of the urine collection container (214) and the second conduit, or at least proximate to an exhaust of the pump.

7. The portable urine collection system of any of claims 1-6 further comprising a sensor (215) secured to one of the urine collection container (214) or the container support (302) and configured to detect a property relating at least to a volume of the urine in the urine collection container.

8. The portable urine collection system of claim 7, wherein the sensor (215) includes a weight sensor secured to the container support (302) and configured to detect a weight of the volume of the urine collected in the urine collection container (214).

9. The portable urine collection system of claim 7, wherein the sensor (215) includes a level sensor secured to one of the urine collection container (214) or the container support (302) and configured to detect a level of the volume of the urine collected in the urine collection container.

10. The portable urine collection system of any of claims 7-9, further comprising a controller (321) configured to communicate with the sensor (215), wherein the sensor is configured communicate an alert to the controller when the volume of the urine in the urine collection container (214) has reached or exceeded a predetermined volume and the controller is configured to coordinate a user alert to notify the user or a caregiver that the volume of the urine in the urine collection container has reached or exceeded the predetermined volume, wherein the user alert coordinated by the controller includes at least one of a light, a noise, or a haptic feedback.

11. The portable urine collection system of claim 10, wherein the controller (321) is configured to coordinate the user alert to notify the user or the caregiver that the volume of the urine in the urine collection container (214) has reached or exceeded two or more of 50% of a maximum volume of the urine collection container, 75% of the maximum volume of the urine collection container, and/or 90% of the maximum volume of the urine collection container.

12. The portable urine collection system of any of claims 10 or 11 wherein the controller (321) is configured to coordinate at least one of a filter replacement alert indicating replacement of the one or more activated carbon and zeolite filters (218) is suggested, a battery charging alert indicating charging of the rechargeable battery (308) is suggested, a battery replacement alert indicating replacement of the rechargeable battery is suggested, a solar panel alert indicating performance of the solar panel (306), or a position alert indicating a change of position by the user is suggested.

13. The portable urine collection system of any of claims 10-12, wherein the controller (321) is secured to an arm of the motorized wheelchair (300) and includes a touch screen.

14. A method of assembling the portable urine collection system according to any of the previous claims, the method comprising:
detachably securing the container support (302) to the back of the motorized wheelchair (300) that is adjustable between a sitting orientation, a standing orientation, and a reclined orientation, the container support including the one or more sidewalls and the lid (219) adjustable between an open position and a closed position;
with the lid in the open position, inserting the urine collection container (214) and the pump (216) in fluid communication with an interior region of the urine collection container in the container support;
positioning the urine collection device (212) proximate to a urethra of a user; and
attaching the first conduit (217a) to the port or adapter on the container support (302) on the outside of the container support (302) to fluidly couple the urine collection device to the urine collection container with the first conduit (217a), wherein the pump is configured to pull an at least partial vacuum on the interior region of the urine collection container effective to draw urine from the urine collection device through the first conduit into the urine collection container and draw urine from the urine collection device when the motorized wheelchair is in each of the sitting orientation, the standing orientation, and the reclined orientation.

## Patentansprüche

1. Tragbares Urinsammelsystem, umfassend:
einen motorisierten Rollstuhl (300);
eine Urinsammelvorrichtung (212), die dazu eingerichtet ist, zumindest nahe einer Harnröhre eines Benutzers positioniert zu werden;
eine erste Leitung (217a), die in Fluidverbindung mit der Urinsammelvorrichtung steht;
einen Urinsammelbehälter (214), der einen Innenbereich aufweist;
eine Pumpe (216), die in Fluidverbindung mit dem Urinsammelbehälter steht und dazu eingerichtet ist, in dem Innenbereich des Urinsammelbehälters zumindest ein Teilvakuum zu erzeugen, das wirksam ist, um Urin von der Urinsammelvorrichtung durch die erste Leitung in den Urinsammelbehälter zu ziehen; und
einen Behälterträger (302), der eine oder mehrere Seitenwände und einen Deckel umfasst, der zwischen einer offenen Position und einer geschlossenen Position verstellbar ist, wobei der Behälterträger mechanisch an einer Rückseite des motorisierten Rollstuhls (300) gesichert ist, um den Behälterträger lösbar an dem motorisierten Rollstuhl zu sichern, wobei der Behälterträger derart bemessen und dimensioniert ist, dass er den Urinsammelbehälter und die Pumpe darin trägt, und wobei die Pumpe dazu eingerichtet ist, den Urin von der Urinsammelvorrichtung zu ziehen, wenn sich der motorisierte Rollstuhl jeweils in der Sitzausrichtung, der Stehausrichtung und der zurückgelehnten Ausrichtung befindet;
wobei das tragbare Urinsammelsystem **dadurch gekennzeichnet ist, dass** der motorisierte Rollstuhl zwischen einer Sitzausrichtung, einer Stehausrichtung und einer zurückgelehnten Ausrichtung verstellbar ist; wobei der Behälterträger (302) einen Anschluss oder Adapter umfasst, der dazu eingerichtet ist, an der ersten Leitung (217a) angebracht oder gesichert zu werden; wobei das Urinsammelsystem eine zusätzliche Leitung umfasst, die in dem Behälterträger (302) positioniert ist und eine Fluidverbindung zwischen der ersten Leitung (217a) und dem Urinsammelbehälter (214) bereitstellt; wobei die erste Leitung (217a) an dem Anschluss oder Adapter an dem Behälterträger (302) auf der Außenseite des Behälterträgers (302) angebracht ist und die zusätzliche Leitung an dem Anschluss oder Adapter an dem Behälterträger auf der Innenseite des Behälterträgers (302) angebracht ist, wobei der Anschluss oder Adapter eine Fluidverbindung zwischen der ersten Leitung (217a) und der in dem Behälterträger (302) aufgenommenen zusätzlichen Leitung bereitstellt.

2. Tragbares Urinsammelsystem nach Anspruch 1, wobei der Behälterträger (302) ein oder mehrere Schalldämpfungselemente umfasst, die an einer Innenfläche der einen oder mehreren Seitenwände des Behälterträgers angeordnet sind, wobei das eine oder die mehreren Schalldämpfungselemente mindestens eines von Schaumstoff oder schalldämpfendem Gewebe umfassen.

3. Tragbares Urinsammelsystem nach einem der Ansprüche 1 bis 2, wobei der Deckel (219) des Behälterträgers (302) einen Verriegelungsmechanismus umfasst, der dazu eingerichtet ist, den Deckel selektiv in der geschlossenen Position zu halten.

4. Tragbares Urinsammelsystem nach einem der Ansprüche 1 bis 3, ferner umfassend:
eine wiederaufladbare Batterie (308), die elektrisch mit der Pumpe (216) gekoppelt und dazu eingerichtet ist, die Pumpe mit Energie zu versorgen;
ein Solarpanel (306), das an einem äußeren Abschnitt des Behälterträgers (302) gesichert und elektrisch mit der wiederaufladbaren Batterie gekoppelt ist, wobei das Solarpanel dazu eingerichtet ist, die wiederaufladbare Batterie zumindest teilweise wiederaufzuladen; und
ein Kabel (304), das elektrisch mit der wiederaufladbaren Batterie gekoppelt ist und mit einer oder mehreren von einer Energiequelle des motorisierten Rollstuhls oder einer Steckdose gekoppelt ist, um die wiederaufladbare Batterie wiederaufzuladen oder die Pumpe mit Energie zu versorgen.

5. Tragbares Urinsammelsystem nach einem der Ansprüche 1 bis 3, ferner umfassend ein Kabel (304), das elektrisch mit der Pumpe (216) und mit einer Batterie oder einer Energiequelle (308) des motorisierten Rollstuhls (300) gekoppelt ist, um die Pumpe mit Energie zu versorgen.

6. Tragbares Urinsammelsystem nach einem der Ansprüche 1 bis 5, ferner umfassend:
eine zweite Leitung (217b), die eine Fluidverbindung zwischen dem Urinsammelbehälter (214) und der Pumpe (216) bereitstellt; und
einen oder mehrere Aktivkohle- und Zeolithfilter (218), die dazu eingerichtet sind, Gerüche aus hindurchströmender Luft zu entfernen, wobei die eine oder
mehreren Aktivkohlen in der zweiten Leitung, zwischen dem Innenbereich des Urinsammelbehälters (214) und der zweiten Leitung oder zumindest nahe einem Auslass der Pumpe positioniert sind.

7. Tragbares Urinsammelsystem nach einem der Ansprüche 1 bis 6, ferner umfassend einen Sensor (215), der an einem von dem Urinsammelbehälter (214) oder dem Behälterträger (302) gesichert und dazu eingerichtet ist, eine Eigenschaft zu erfassen, die sich zumindest auf ein Volumen des Urins in dem Urinsammelbehälter bezieht.

8. Tragbares Urinsammelsystem nach Anspruch 7, wobei der Sensor (215) einen Gewichtssensor umfasst, der an dem Behälterträger (302) gesichert und dazu eingerichtet ist, ein Gewicht des Volumens des in dem Urinsammelbehälter (214) gesammelten Urins zu erfassen.

9. Tragbares Urinsammelsystem nach Anspruch 7, wobei der Sensor (215) einen Füllstandssensor umfasst, der an einem von dem Urinsammelbehälter (214) oder dem Behälterträger (302) gesichert und dazu eingerichtet ist, einen Füllstand des Volumens des in dem Urinsammelbehälter gesammelten Urins zu erfassen.

10. Tragbares Urinsammelsystem nach einem der Ansprüche 7 bis 9, ferner umfassend eine Steuerung (321), die dazu eingerichtet ist, mit dem Sensor (215) zu kommunizieren, wobei der Sensor dazu eingerichtet ist, eine Warnung an die Steuerung zu kommunizieren, wenn das Volumen des Urins in dem Urinsammelbehälter (214) ein vorbestimmtes Volumen erreicht oder überschritten hat, und wobei die Steuerung dazu eingerichtet ist, eine Benutzerwarnung zu koordinieren, um den Benutzer oder eine Pflegeperson darüber zu benachrichtigen, dass das Volumen des Urins in dem Urinsammelbehälter das vorbestimmte Volumen erreicht oder überschritten hat, wobei die durch die Steuerung koordinierte Benutzerwarnung mindestens eines von einem Licht, einem Geräusch oder einer haptischen Rückmeldung umfasst.

11. Tragbares Urinsammelsystem nach Anspruch 10, wobei die Steuerung (321) dazu eingerichtet ist, die Benutzerwarnung zu koordinieren, um den Benutzer oder die Pflegeperson darüber zu benachrichtigen, dass das Volumen des Urins in dem Urinsammelbehälter (214) zwei oder mehr von 50 % eines Maximalvolumens des Urinsammelbehälters, 75 % des Maximalvolumens des Urinsammelbehälters und/oder 90 % des Maximalvolumens des Urinsammelbehälters erreicht oder überschritten hat.

12. Tragbares Urinsammelsystem nach einem der Ansprüche 10 oder 11, wobei die Steuerung (321) dazu eingerichtet ist, mindestens eines zu koordinieren von einer Filterwechselwarnung, die angibt, dass ein Austausch des einen oder der mehreren Aktivkohle- und Zeolithfilter (218) empfohlen wird, einer Batterieladewarnung, die angibt, dass ein Laden der wiederaufladbaren Batterie (308) empfohlen wird, einer Batteriewechselwarnung, die angibt, dass ein Austausch der wiederaufladbaren Batterie empfohlen wird, einer Solarpanelwarnung, die eine Leistung des Solarpanels (306) angibt, oder einer Positionswarnung, die angibt, dass eine Positionsänderung durch den Benutzer empfohlen wird.

13. Tragbares Urinsammelsystem nach einem der Ansprüche 10 bis 12, wobei die Steuerung (321) an einem Arm des motorisierten Rollstuhls (300) gesichert ist und einen Berührungsbildschirm umfasst.

14. Verfahren zum Zusammenbauen des tragbaren Urinsammelsystems nach einem der vorhergehenden Ansprüche, wobei das Verfahren umfasst:
lösbares Sichern des Behälterträgers (302) an der Rückseite des motorisierten Rollstuhls (300), der zwischen einer Sitzausrichtung, einer Stehausrichtung und
einer zurückgelehnten Ausrichtung verstellbar ist, wobei der Behälterträger die eine oder mehreren Seitenwände und den Deckel (219) umfasst, der zwischen einer offenen Position und einer geschlossenen Position verstellbar ist;
bei in der offenen Position befindlichem Deckel, Einsetzen des Urinsammelbehälters (214) und der Pumpe (216), die in Fluidverbindung mit einem Innenbereich des Urinsammelbehälters steht, in den Behälterträger;
Positionieren der Urinsammelvorrichtung (212) nahe einer Harnröhre eines Benutzers; und
Anbringen der ersten Leitung (217a) an dem Anschluss oder Adapter an dem Behälterträger (302) auf der Außenseite des Behälterträgers (302), um die Urinsammelvorrichtung mit der ersten Leitung (217a) fluidisch mit dem Urinsammelbehälter zu koppeln, wobei die Pumpe dazu eingerichtet ist, in dem Innenbereich des Urinsammelbehälters zumindest ein Teilvakuum zu erzeugen, das wirksam ist, um Urin von der Urinsammelvorrichtung durch die erste Leitung in den Urinsammelbehälter zu ziehen und Urin von der Urinsammelvorrichtung zu ziehen, wenn sich der motorisierte Rollstuhl jeweils in der Sitzausrichtung, der Stehausrichtung und der zurückgelehnten Ausrichtung befindet.

## Revendications

1. Système portable de collecte d'urine, comprenant :
un fauteuil roulant motorisé (300) ;
un dispositif de collecte d'urine (212) configuré pour être positionné au moins à proximité d'un urètre d'un utilisateur ;
un premier conduit (217a) en communication fluidique avec le dispositif de collecte d'urine ;
un récipient de collecte d'urine (214) présentant une région intérieure ;
une pompe (216) en communication fluidique avec le récipient de collecte d'urine et configurée pour créer un vide au moins partiel dans la région intérieure du récipient de collecte d'urine, de manière à aspirer l'urine du dispositif de collecte d'urine à travers le premier conduit jusque dans le récipient de collecte d'urine ; et
un support de récipient (302) comprenant une ou plusieurs parois latérales et un couvercle réglable entre une position ouverte et une position fermée, le support de récipient étant fixé mécaniquement à l'arrière du fauteuil roulant motorisé (300) afin de fixer de manière amovible le support de récipient au fauteuil roulant motorisé, dans lequel le support de récipient est dimensionné pour supporter en son sein le récipient de collecte d'urine et la pompe, et la pompe est configurée pour aspirer l'urine du dispositif de collecte d'urine lorsque le fauteuil roulant motorisé se trouve dans chacune de l'orientation assise, de l'orientation debout et de l'orientation inclinée ;
ledit système portable de collecte d'urine étant **caractérisé en ce que** ledit fauteuil roulant motorisé est réglable entre une orientation assise, une orientation debout et une orientation inclinée ; le support de récipient (302) comprend un orifice ou un adaptateur configuré pour être raccordé ou fixé au premier conduit (217a) ; le système de collecte d'urine comprend un conduit supplémentaire positionné dans le support de récipient (302), assurant une communication fluidique entre le premier conduit (217a) et le récipient de collecte d'urine (214) ; le premier conduit (217a) est raccordé à l'orifice ou à l'adaptateur situé sur le support de récipient (302), du côté extérieur du support de récipient (302), et le conduit supplémentaire est raccordé à l'orifice ou à l'adaptateur situé sur le support de récipient, du côté intérieur du support de récipient (302), l'orifice ou l'adaptateur assurant une communication fluidique entre le premier conduit (217a) et le conduit supplémentaire logé dans le support de récipient (302).

2. Système portable de collecte d'urine selon la revendication 1, dans lequel le support de récipient (302) comprend un ou plusieurs éléments d'atténuation acoustique disposés sur une surface intérieure de la ou des parois latérales du support de récipient, le ou les éléments d'atténuation acoustique comprenant au moins l'un des éléments parmi une mousse ou un tissu d'atténuation acoustique.

3. Système portable de collecte d'urine selon l'une quelconque des revendications 1-2, dans lequel le couvercle (219) du support de récipient (302) comprend un mécanisme de verrouillage configuré pour retenir sélectivement le couvercle en position fermée.

4. Système portable de collecte d'urine selon l'une quelconque des revendications 1-3, comprenant en outre :
une batterie rechargeable (308) couplée électriquement à la pompe (216) et configurée pour alimenter la pompe ;
un panneau solaire (306) fixé à une partie extérieure du support de récipient (302) et couplé électriquement à la batterie rechargeable, le panneau solaire étant configuré pour recharger au moins partiellement la batterie rechargeable ; et
un câble (304) couplé électriquement à la batterie rechargeable et couplé à un ou plusieurs éléments parmi une source d'alimentation du fauteuil roulant motorisé ou une prise électrique, afin de recharger la batterie rechargeable ou d'alimenter la pompe.

5. Système portable de collecte d'urine selon l'une quelconque des revendications 1-3, comprenant en outre un câble (304) couplé électriquement à la pompe (216) et à une batterie ou à une source d'alimentation (308) du fauteuil roulant motorisé (300), afin d'alimenter la pompe.

6. Système portable de collecte d'urine selon l'une quelconque des revendications 1-5, comprenant en outre :
un deuxième conduit (217b) assurant une communication fluidique entre le récipient de collecte d'urine (214) et la pompe (216) ; et
un ou plusieurs filtres à charbon actif et à zéolite (218) configurés pour éliminer les odeurs de l'air qui les traverse, le ou les charbons actifs étant positionnés dans le deuxième conduit, entre la région intérieure du récipient de collecte d'urine (214) et le deuxième conduit, ou au moins à proximité d'un échappement de la pompe.

7. Système portable de collecte d'urine selon l'une quelconque des revendications 1-6, comprenant en outre un capteur (215) fixé au récipient de collecte d'urine (214) ou au support de récipient (302), et configuré pour détecter une propriété relative au moins à un volume d'urine dans le récipient de collecte d'urine.

8. Système portable de collecte d'urine selon la revendication 7, dans lequel le capteur (215) comprend un capteur de poids fixé au support de récipient (302) et configuré pour détecter un poids du volume d'urine collecté dans le récipient de collecte d'urine (214).

9. Système portable de collecte d'urine selon la revendication 7, dans lequel le capteur (215) comprend un capteur de niveau fixé au récipient de collecte d'urine (214) ou au support de récipient (302), et configuré pour détecter un niveau du volume d'urine collecté dans le récipient de collecte d'urine.

10. Système portable de collecte d'urine selon l'une quelconque des revendications 7-9, comprenant en outre un dispositif de commande (321) configuré pour communiquer avec le capteur (215), dans lequel le capteur est configuré pour communiquer une alerte au dispositif de commande lorsque le volume d'urine dans le récipient de collecte d'urine (214) a atteint ou dépassé un volume prédéterminé, et le dispositif de commande est configuré pour coordonner une alerte utilisateur afin de notifier à l'utilisateur ou à un aidant que le volume d'urine dans le récipient de collecte d'urine a atteint ou dépassé le volume prédéterminé, dans lequel l'alerte utilisateur coordonnée par le dispositif de commande comprend au moins l'un des éléments parmi une lumière, un bruit ou un retour haptique.

11. Système portable de collecte d'urine selon la revendication 10, dans lequel le dispositif de commande (321) est configuré pour coordonner l'alerte utilisateur afin de notifier à l'utilisateur ou à l'aidant que le volume d'urine dans le récipient de collecte d'urine (214) a atteint ou dépassé au moins deux parmi 50 % d'un volume maximal du récipient de collecte d'urine, 75 % du volume maximal du récipient de collecte d'urine et/ou 90 % du volume maximal du récipient de collecte d'urine.

12. Système portable de collecte d'urine selon l'une quelconque des revendications 10 ou 11, dans lequel le dispositif de commande (321) est configuré pour coordonner au moins l'un des éléments parmi une alerte de remplacement de filtre indiquant que le remplacement du ou des filtres à charbon actif et à zéolite (218) est suggéré, une alerte de charge de batterie indiquant que la charge de la batterie rechargeable (308) est suggérée, une alerte de remplacement de batterie indiquant que le remplacement de la batterie rechargeable est suggéré, une alerte de panneau solaire indiquant les performances du panneau solaire (306), ou une alerte de position indiquant qu'un changement de position par l'utilisateur est suggéré.

13. Système portable de collecte d'urine selon l'une quelconque des revendications 10-12, dans lequel le dispositif de commande (321) est fixé à un bras du fauteuil roulant motorisé (300) et comprend un écran tactile.

14. Procédé d'assemblage du système portable de collecte d'urine selon l'une quelconque des revendications précédentes, le procédé comprenant les étapes consistant à :
fixer de manière amovible le support de récipient (302) à l'arrière du fauteuil roulant motorisé (300), lequel est réglable entre une orientation assise, une orientation debout et une orientation inclinée, le support de récipient comprenant la ou les parois latérales et le couvercle (219) réglable entre une position ouverte et une position fermée ;
le couvercle étant en position ouverte, insérer, dans le support de récipient, le récipient de collecte d'urine (214) et la pompe (216) en communication fluidique avec une région intérieure du récipient de collecte d'urine ;
positionner le dispositif de collecte d'urine (212) à proximité d'un urètre d'un utilisateur ; et
raccorder le premier conduit (217a) à l'orifice ou à l'adaptateur situé sur le support de récipient (302), du côté extérieur du support de récipient (302), afin de coupler fluidiquement le dispositif de collecte d'urine au récipient de collecte d'urine au moyen du premier conduit (217a), dans lequel la pompe est configurée pour créer un vide au moins partiel dans la région intérieure du récipient de collecte d'urine, de manière à aspirer l'urine du dispositif de collecte d'urine à travers le premier conduit jusque dans le récipient de collecte d'urine et à aspirer l'urine du dispositif de collecte d'urine lorsque le fauteuil roulant motorisé se trouve dans chacune de l'orientation assise, de l'orientation debout et de l'orientation inclinée.
